# EUROPÄISCHE PATENTSCHRIFT

(11) **EP 1 307 413 B1**
(45) Veröffentlichungstag und Bekanntmachung des Hinweises auf die Patenterteilung: **27.04.2005**
(21) Anmeldenummer: 01967245.0
(22) Anmeldetag: 01.08.2001
(51) Int. Cl.: C07B 37/04, C07C 13/72, C07C 33/28

(54) **VERFAHREN ZUR HERSTELLUNG VON OLEFINSUBSTITUIERTEN AROMATEN ODER HETEROAROMATEN**
METHOD FOR PRODUCING OLEFIN-SUBSTITUTED AROMATIC OR HETEROAROMATIC COMPOUNDS
PROCEDE DE PRODUCTION DE COMPOSES AROMATIQUES OU HETEROAROMATIQUES A SUBSTITUTION OLEFINIQUE

(30) Priorität: 01.08.2000 DE 10037390
(43) Veröffentlichungstag der Anmeldung: 07.05.2003
(73) Patentinhaber: Covion Organic Semiconductors GmbH, 65926 Frankfurt (DE)
(72) Erfinder: SPREITZER, Hubert, 68519 Viernheim (DE); STÖSSEL, Philipp, 65929 Frankfurt (DE); BECKER, Heinrich, 61479 Glashütten (DE)
(74) Vertreter: Luderschmidt, Schüler & Partner GbR
(86) Internationale Anmeldenummer: PCT/EP2001/009175
(87) Internationale Veröffentlichungsnummer: WO 2002/010093

(56) Entgegenhaltungen:
- EP-A- 0 105 282
- DE-A- 19 843 012
- US-A- 4 400 544
- REETZ M T ET AL: "A Highly Active Phosphine-free Catalyst System for Heck Reactions of Aryl Bromides" TETRAHEDRON LETTERS, ELSEVIER SCIENCE PUBLISHERS, AMSTERDAM, NL, Bd. 39, Nr. 46, 12. November 1998 (1998-11-12), Seiten 8449-8452, XP004139440 ISSN: 0040-4039

## Beschreibung

Aromatische oder heteroaromatische Verbindungen, sowohl niedermolekular als auch hochmolekular, die mit Olefinen substitutiert sind, spielen eine bedeutende Rolle in der Industrie, so als Hilfsmittel bzw. Additive in Kosmetikzubereitungen, wie zum Beispiel in Sonnenschutzmitteln, diversen Fein- und Elektronikchemikalien, Vorprodukten für Pharmaka und Agrochemikalien, um nur einige Anwendungsfelder zu nennen. Vor allem auch in dem stark wachsenden Feld für organische Halbleiter (z. B. Anwendungen in organischen bzw. polymeren Leuchtdioden, organischen Solarzellen, organischen ICs) sind gerade diese Verbindungen von herausragender Bedeutung.

Für die Darstellung sind verschiedenartigste Alternativen bekannt, die jedoch nicht für alle Fälle eine - z. B. ökonomisch und ökologisch - befriedigende Lösung anbieten. Bei vielen Verfahren fallen Koppelprodukte an, die abgetrennt und aufwendig entsorgt werden müssen.

Vom synthetischen Aspekt her, ist häufig eine direkte Verknüpfung eines Aromaten/Heteroaromatenderivates mit einem Olefinderivat ein günstiger Ausgangspunkt.
Hier ist wiederum die sogenannte Heck-Reaktion (vgl. R. F. Heck, Compr. Org. Synth. **1991,** 883) eine geeignete Wahl (vgl. Figur 1). 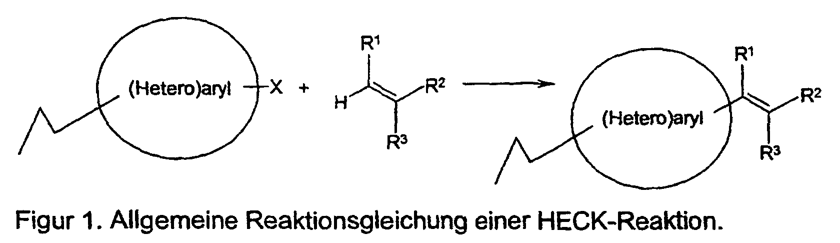 Obwohl diese Reaktion schon seit etlichen Jahren erforscht wird, mangelt es immer noch an einem industriell tauglichen Verfahren. Erste Ansätze in diese Richtung wurden jetzt kürzlich von der Arbeitsgruppe um Prof. Reetz gemacht. Da diese Arbeiten (vgl. unten: WO98/42644, DE 198 43 012) den nächstliegenden Stand der Technik zu der vorliegenden Anmeldung ausmachen, werden diese hiermit via Zitat als Bestandteil der Anmeldung betrachtet. Es wird vor allem darauf verzichtet, die dort ausführlichen Beschreibungen zum allgemeinen Stand der Technik bezüglich HECK-Reaktionen zu wiederholen.
Reetz konnte in WO98/42644 zeigen, daß die Umsetzung einer entsprechenden aromatischen oder heteroaromatischen Verbindung mit einem entsprechenden Olefin in Gegenwart eines Palladiumkatalysators, eines Tetraarylphosphoniumsalzes, eines dipolar aprotischen Lösemittels, einer Base und gegebenenfalls eines Additives (welches ein α- oder β-Aminosäurederivat darstellt) mit deutlich besserer Ausbeute verläuft als dies dem in der o. g. Anmeldung zitierte Stand der Technik zu entnehmen ist. So können unter industrietauglichen Bedingungen Umsatzgrade von bis zu 98% und Kopplungsselektivitäten von ebenfalls bis zu ca. 98% erreicht werden (siehe Beispiel 24 der o. g. Anmeldung). Selektivität im Sinne dieser Anmeldung bedeutet, daß die gewünschte Stereoselektivität erzielt wird. Dies ist bei Reetz bei den Umsetzungen mit Styrol meist durch die Angabe der drei möglichen Produkte (*trans*-Produkt, *cis*-Produkt und 2-verknüpftes Produkt) gekennzeichnet. Bei bestimmten Olefinen wird die Selektivität 100% erreicht, wenn nur eine Verknüpfungsmöglichkeit besteht. In einer weiteren Verbesserung (DE 198 43 012) konnte Reetz zeigen, daß für bestimmte Aryl/Heteroarylderivate ein noch einfacheres Verfahren zu sehr guten Ergebnissen führt: verzichtet man auf die Tetraarylphosphoniumsalze und verwendet dafür konsequent α- oder β-Aminosäurederivate, bevorzugt Dimethylglycin, werden technisch verwertbare Umsätze von bis zu 99% und Selektivtäten bis zu ca. 99% (vgl. v. a. Beispiele 39 bis 45 in DE 198 43 012) erreicht. Unbefriedigend ist jedoch, daß ein sehr hoher Umsatz nicht gleichzeitig auch noch sehr hohe Selektivitäten ergibt. Vorteil dieses letztgenannten Verfahrens ist in jedem Fall der Verzicht auf Phosphin/Phosphoniumkomponenten, die sonst teilweise mühevoll aus dem Produkt entfernt werden müssen.
Für viele einfache Umsetzungen könnten die durch Reetz gefundenen und publizierten Bedingungen ausreichen, um ökonomisch sinnvolle Verfahren zu entwickeln. Stellt sich jedoch das Problem, eine multifunktionelle Verbindung umzusetzen (vgl. Figur 2) oder gar eine Polymerisation (vgl. Figur 3) auf diesem Wege aufzubauen, können selbst Umsatzgrade bis zu 99% (bei dann deutlich unter 100% liegenden Selektivitäten) zu deutlichen Problemen führen.

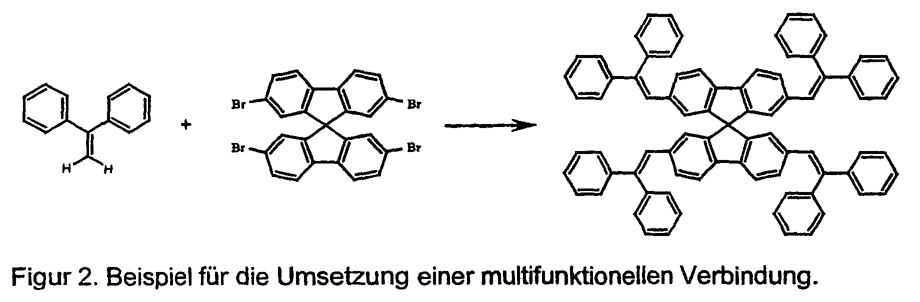

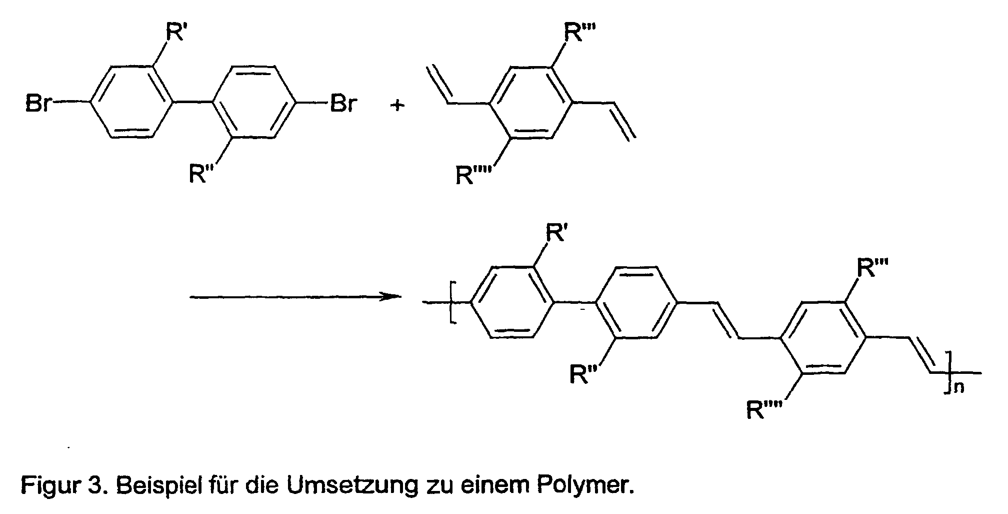

So führt beim Beispiel aus Figur 2 eine beispielhafte Umsatzrate (pro Einzelschritt) von 99% mit einer jeweiligen SELEKTIVITÄT von angenommenen 99% (d. h. pro Einzeladdition 0.99 x 0.99 = 98% Ausbeute an gewünschtem Substitutionsmuster) zu einer Gesamtausbeute von ca. 92%. Diese relativ hohe Ausbeute ist jedoch angesichts von Reinheitsanforderungen von +99.9% in z. B. elektronischen Anwendungen immer noch unbefriedigend, da die Aufreinigung durch die hohe Zahl verschiedener Nebenkomponenten sehr aufwendig ist. Damit ist klar, daß die Anwendung der Reetz'schen Verfahren hierfür unbefriedigend ist (vgl. auch die Beispiele 4 bis 7 in diesem Zusammenhang).

Analoges gilt für das Polymerisationsbeispiel in Figur 3. Eine Umsatzrate von 99% führt bei einer Polykondensation gemäß Theorie zu einem maximalen Polymerisationsgrad von 100. Des weiteren führt hier eine Selektivität von (deutlich) weniger als 100% entweder zu Defekten in der Polymerhauptkette oder zu weiteren Kettenabbrüchen. In der Realität dürfte daher der Polymerisationsgrad eher noch deutlich unter 100 liegen. Dies zusammen mit den möglichen Hauptkettendefekten kann sich jedoch für viele Anwendungen (zum Beispiel Verwendung in polymeren Leuchtdioden oder in organischen ICs) als schwerwiegendes Problem erweisen.

Da nun offensichtlich ist, daß die oben aufgezeigte HECK-Reaktion einerseits sehr gut zur Darstellung entsprechender olefinsubstituierter Aromaten oder Heteroaromaten prinzipiell geeignet ist, andererseits die bisherigen Methoden aber für bestimmte Belange noch unzureichend sind, besteht ein deutlicher Bedarf an Verbesserung des oben gezeigten Stands der Technik.

EP-A 0 105 282 ist Stand der Technik gemäß Artikel 54(3) EPÜ und beschreibt die so genannte Heck-Reaktion in Gegenwart von Kupfer (I)-Jodid.

Es wurde nun überraschend gefunden, daß die oben im Stand der Technik aufgezeigte HECK-Reaktion durch eine klar definierte Änderung entscheidend verbessert werden kann, so daß dadurch die in der Beschreibung zu den Figuren 2 und 3 aufgezeigten Probleme gelöst werden können. So wurden beispielsweise (vgl. die Beispiele) Produkte aus Umsatz x Selektivtät für Einzelschritte in der Größenordnung mehr als 99.5% erreicht, was auch bei der Umsetzung von multifunktionellen Verbindungen (analog Figur 2) zu Rohausbeuten/ -reinheiten von über 98% führt und Polymerisationsgrade von einigen Hundert bis 1000 ermöglicht. Multifunktionell im Sinne dieser Anmeldung soll bedeuten, daß eine Verbindung mehrere (z. B. zwei, drei, vier, fünf, usw.) gleiche oder gleichartige funktionelle Einheiten enthält, die in der entsprechenden Umsetzung (hier HECK-Reaktion) alle in der gleichen Weise zu einem Produktmolekül reagieren. Mit der Umsetzung von multifunktionellen Verbindungen ist hier zunächst die Umsetzung einer multifunktionellen Verbindung mit mehreren monofunktionellen Verbindungen zu einer definierten "niedermolekularen" Verbindung gemeint. Werden hingegen (mindestens) zwei verschiedene multifunktionelle Verbindungen miteinander in Reaktion gebracht, wird das Produkt polymeren Charakter aufweisen, wie dies in Figur 3 für die Reaktion von zwei bifunktionellen Verbindungen dargestellt ist. Auch dies stellt ausdrücklich eine HECK-Reaktion im Sinne dieser Erfindung dar.

Das neue, erfindungsgemäße Verfahren ist die Umsetzung einer funktionellen Aryloder Heteroarylverbindung mit einem Olefinderivat, weiches mindestens ein H-Atom an der Doppelbindung aufweist, in Gegenwart einer einfachen Palladiumverbindung, gegebenenfalls in der Gegenwart eines stickstoffhaltigen Additives, einer Base in einem Lösungsmittel, unter Bildung einer C-C-Verknüpfung und formaler Abspaltung der funktionellen Gruppe des Aryl- bzw. Heteroarylderivates und eines H-Atoms der Olefinverbindung, gekennzeichnet durch die Anwesenheit von mindestens einem metallhaltigen Additiv, das von Palladium verschieden ist, in einer Menge zwischen 1 und 500 mol% (bezogen auf die Palladiummenge).

Bevorzugt ist das o. g. Verfahren, wenn tatsächlich neben dem metallhaltigen Additiv ein stickstoffhaltiges Additiv verwendet wird.
Ganz besonders bevorzugt sind hierbei Aminocarbonsäuren und ihre Derivate, wie Dimethylglycin, 4-Dimethylaminobuttersäure, 3-Indolylessigsäure.

Die metallhaltigen Additive, sind Metalle, Legierungen, Verbindungen, Salze oder Chalkogenide der d-Übergangsgruppen des Periodensystems. Bevorzugt sind hierbei Übergangsmetalle ab der Vanadiumgruppe bis zur Zinkgruppe, besonders bevorzugt die entsprechenden Additive mit den Metallen Eisen, Kobalt, Nickel, Ruthenium, Rhodium, Osmium, Iridium, Platin, ganz besonders bevorzugt Eisen, Nickel und Kobalt, am meisten bevorzugt Eisen. Von den Metallen können Salze, wie Halogenide, Sulfate, Acetate oder weitere Carboxylate eingesetzt werden. Weiterhin ist auch der Einsatz von Oxiden oder Komplexen (wie z. B. Metallocenen) möglich. Es ist aber auch erfindungsgemäß, die Metalle direkt oder als Legierungen dem Reaktionsgemisch zuzuführen, z. B. als Metallstaub oder Pulver. Hier ist bei der besonders bevorzugten Verwendung von Eisen auch der Einsatz von Eisenerzen, verschiedenen Stahl- oder Gußeisenformen (z. B. Pulvern oder Watten) oder ähnlichen handelsüblichen Formen möglich.

Aryl- bzw. Heteroarylverbindungen sind monofunktionelle Aromaten bzw. Heteroaromaten mit 4 bis 40 C-Atomen, welche mit einem oder auch mehreren linearen, verzweigten oder cyclischen Alkyl- bzw. Alkoxyresten mit 1 bis 20 C-Atomen, bei denen wiederum eine oder mehrere CH₂-Gruppen die nicht aufeinander folgen durch O, S, C=O, oder eine Carboxygruppe ersetzt sein können, unsubstituierten C-4 bis C-20 Aryl- oder Heteroarylresten, Fluor, Cyano, Nitro, Sulfonsäurederivaten substituiert sein können bzw. auch unsubstituiert sein können. Hier wird auch ausdrücklich auf die oben zitierten Anmeldungen von Reetz et al. und die darin aufgeführten Aryl- und Heteroarylsysteme verwiesen.
Einfache Verbindungen, die bevorzugt verwendet werden können, sind die entsprechenden funktionalisierten Derivate von Benzol, Naphthalin, Anthracen, Pyren, Biphenyl, Fluoren, Spiro-9,9'-bifluoren, Phenanthren, Triptycen, Pyridin, Furan, Thiophen, Pyrrol, Chinolin, Chinoxalin, Pyrimidin und Pyrazin.
Weiterhin sind ausdrücklich entsprechende (im Sinne des obigen Textes) multifunktionelle Verbindung mit umfaßt, ebenso die bei einer Polymerisation auftretenden Oligomeren mit funktionellen Aryl- bzw. Heteroaryl-Enden.

Die funktionelle Gruppe in den funktionellen Aryl- oder Heteroarylverbindungen ist eine für die beschriebene Umsetzung geeignete reaktive Abgangsgruppe. Dies ist bevorzugt Chlor, Brom, lod, Methylsulfonat, Tosylat, Triflat, Nonaflat bzw. eine Diazoniumsalz-Gruppierung.

Das Olefinderivat ist - analog zu den Beschreibungen in den zitierten Reetz-Anmeldungen - eine maximal dreifach substituierte Doppelbindung. Die Substituenten können analog denen, die für die Aryl- bzw. Heteroarylverbindung beschrieben sind, sein, oder auch Aldehyd-, Carbonsäurederivatgruppen, oder auch weitere Gruppen wie bei Reetz beschrieben, bedeuten.
Einfache Verbindungen, die bevorzugt verwendet werden können, sind Ethylen, Propylen, Acrylsäurederivate, Methacrylsäurederivate, Styrolderivate, Diphenylethenderivate, Vinylether und Vinylacetat.
Weiterhin sind ausdrücklich entsprechende (im Sinne des obigen Textes) multifunktionelle Verbindung mit umfaßt, ebenso die bei einer Polymerisation auftretenden Oligomeren mit Olefinenden.

Die einfache Palladiumverbindung bedeutet eine Palladium-II-verbindung im Sinne der Anmeldungen von Reetz et al., beispielsweise Palladium-II-halogenide, Palladium-II-acetat, oder einfache daraus ableitbare Komplexe bzw. auch disperses oder kolloidales metallisches Palladium geträgert oder ungeträgert oder auch weitere Palladium-(O)-verbindungen wie Pd₂dba₃ und ähnliche.
Des weiteren sind auch folgende Pd-verbindungen einsetzbar:
Palladiumketonate, Palladiumacetylacetonate, Nitrilpalladiumhalogenide, Olefinpalladiumhalogenide, Allylpalladiumhalogenide und Palladiumbiscarboxylate.

Basen werden analog den Anmeldungen von Reetz et al. verwendet, bevorzugt Hydrogencarbonate, Carbonate, Acetate und weitere Carboxylate, wie Propionate, Formiate und Benzoate.
Eine Variante kann hier auch darin bestehen, das metallhaltige Additiv gleich als Teil der Base zu verwenden, z. B. durch die (Mit)verwendung von Eisen-(II)-acetat, oder Nickel-(II)-carbonat.

Lösemittel werden ebenfalls analog zur Anmeldung von Reetz et al. verwendet, d. h. bevorzugt dipolar aprotische Lösemittel, besonders bevorzugt Dimethylacetamid, Dimethylformamid und N-Methyl-pyrrolidinon; gegebenenfalls aber auch protische Lösemittel wie Methanol oder Ethylenglycol.

Das gegebenenfalls bevorzugt mit zu verwendende stickstoffhaltige Additiv ist entweder ebenfalls analog zu den Beschreibungen von Reetz et al., d. h. eine α-oder β-Aminocarbonsäure bzw. abgeleitete Derivate davon, wie zum Beispiel das von Reetz als bevorzugt beschriebene Dimethylglycin, oder auch längere Aminocarbonsäuren, wie γ- oder δ-Aminocarbonsäuren und deren Derivate, wie z. B. 4-Dimethylaminobuttersäure oder 3-Indolylessigsäure.

Bei dem erfindungsgemäßen Verfahren wird der Palladiumkatalysator in der Regel in einer Menge von 0.001 bis 10mol% (Palladium) bezogen auf die Menge zu schließender C-C-Verknüpfungen eingesetzt. Bevorzugt ist hier der Bereich von 0.01% bis 5%, besonders bevorzugt der Bereich von 0.05% bis 2.5%.

Die Menge des metallhaltigen Additivs im Verhältnis zum Palladiumkatalysator ist relativ unkritisch, ist bezogen auf die Palladiummenge!, bevorzugt zwischen 5 und 200mol%, besonders bevorzugt zwischen 10 und 100mol%.

Die Base wird in der Regel zwischen 0.5 und 10 Äquivalenten, bezogen auf die Menge zu schließender C-C-Verknüpfungen eingesetzt. Bevorzugt ist hier der Bereich zwischen 0.8 und 5 Äquivalenten, besonders bevorzugt zwischen 0.8 bis 3 Äquivalenten.

Die Konzentration der Reaktanden im Lösemittel hängt natürlich von der speziellen Reaktion ab. Bevorzugt findet die Reaktion jedoch im Bereich von 0.1 mol/l bis zu 5 mol/l bezogen auf auf die Menge zu schließender C-C-Verknüpfungen statt. Es ist aber auch möglich, einen der Reaktanden (z. B. die Olefinkomponente) im Überschuß auch direkt als Lösemittel zu verwenden.

Die erfindungsgemäße Reaktion ist thermisch aktiviert und findet daher in der Regel im Temperaturbereich oberhalb Raumtemperatur statt, bevorzugt von 40 bis 200°C, besonders bevorzugt von 60 bis 180°C, ganz besonders bevorzugt von 100 bis 160°C.

Besonders bevorzugt ist das erfindungsgemäße Verfahren, wenn als Palladiumverbindung Palladium-acetat oder -chlorid, und als metallhaltiges Additiv ein Eisen-II-oder III-salz wie Eisenchlorid eingesetzt wird.
Bevorzugt wird das erfindungsgemäße Verfahren zur Umsetzung multifunktioneller Aryl- bzw. Heteroarylverbindungen mit monofunktionell reagierenden Olefinen eingesetzt.

Ebenso bevorzugt wird das erfindungsgemäße Verfahren zur Umsetzung monofunktioneller Aryl- bzw. Heteroarylverbindungen mit multifunktionell reagierenden Olefinen bzw. Verbindungen, die mehrere monofunktionelle Olefin-Enden aufweisen, eingesetzt.

Weiterhin bevorzugt wird das erfindungsgemäße Verfahren zur Herstellung von Polymeren durch die Umsetzung von bifunktionellen Aryl- bzw. Heteroarylverbindungen mit bifunktionellen Olefinderivaten (eventuell Bisolefinen analog zum Beispiel in Figur 3) verwendet.

Diese Bevorzugung ergibt sich einfach aus der hohen Umsetzungsrate und Selektivität des erfindungsgemäßen Verfahrens.

Trotz dieser Bevorzugung für Mehrfach-Umsetzungen, erzielt das erfindungsgemäße Verfahren natürlich gerade auch bei Einfach-Umsetzungen hervorragende Resultate, so daß auch hier das erfindungsgemäße Verfahren bevorzugt angewendet werden kann.

Die beschriebene Erfindung wird durch die nachfolgend aufgeführten Beispiele erläutert, ist aber keinesfalls auf diese beschränkt, sondern kann durch den Fachmann natürlich einfach auf die oben aufgezeigten bzw. in der zitierten Literatur beschriebenen Systeme übertragen werden.

### Beispiel 1:

In einen Reaktionsgefäß mit Septumkappe und Druckausgleichsventil wurden 632.0 mg (1.0 mmol) 2,2',7,7'-Tetrabromspirobifluoren, 9.0 mg (0.04 mmol) Palladium(II)-acetat, 82.5 mg (0.8 mmol) N,N-Dimethylglycin und 1008.1 mg (12.0 mmol) Natriumhydrogencarbonat eingewogen. Zusätzlich wurde die angegebene Menge des metallhaltigen Additivs (s. Tabelle 1 ) zugewogen. Das Reaktionsgefäß wurde dreimal evakuiert und mit Argon geflutet. Anschließend wurden 1081 mg entsprechend 1059 µl (6.0 mmol) 1,1-Diphenylethen und 5.0 ml entgastes N-Methylpyrrolidinon mit Hilfe einer Spritze zugegeben.
Das Reaktionsgefäß wurde verschlossen und das Gemisch 18 h bei der angegebenen Temperatur gerührt.
Nach 18 Stunden wurden jeweils 25 µl Proben entnommen, mit 10 ml Tetrahydrofuran und 10 ml Methanol verdünnt, über einen Spritzenfilter filtriert. Der Gehalt an Heck-Produkt wird via HPLC untersucht.

**Tabelle 1:**

| Beispiel Nr. | Metallhaltiges Additiv, [mol %] bez. auf eingesetztes Palladium | Reaktionstemperatur | Ausbeute Heck-Produkt in der Reaktionsmischung |
|---|---|---|---|
| 1.1 | ― ohne ― | 140° C | 94.1 % |
| 1.2 | Vanadium(III)-acetylacetonat 20 mol % | 140° C | 97.2 % |
| 1.3 | Chrom(III)-acetylacetonat 20 mol % | 140°C | 96.8 % |
| 1.4 | Chrom(III)-acetylacetonat 200 mol % | 140° C | 96.8 % |
| 1.5 | Eisen(II)-acetat 20 mol % | 140° C | 97.9 % |
| 1.6 | Eisen(III)-acetylacetonat 10 mol % | 140° C | 97.7 % |
| 1.7 | Eisen(III)-acetylacetonat 100 mol % | 140° C | 97.7 % |
| 1.8 | Eisen(III)-chlorid Hexahydrat 10 mol % | 140°C | 98.1 % |
| 1.9 | Eisen(III)-chlorid Hexahydrat 20 mol % | 140°C | 98.4 % |
| 1.9 | Eisen(III)-chlorid Hexahydrat 200 mol % | 140°C | 98.0 % |
| 1.10 | Nickel(II)-chlorid Hexahydrat 20 mol % | 140°C | 97.6 % |
| 1.11 | Nickel(II)-chlorid Hexahydrat 100 mol % | 140°C | 97.3 % |

### Beispiel 2:

Durchführung wie in Beispiel 1 beschrieben, wobei N,N-Dimethylglycin durch 134.1 mg (0.8 mmol) 4-(Dimethylamino)-buttersäure Hydrochlorid ersetzt wird.

**Tabelle 2:**

| Beispiel Nr. | Metallhaltiges Additiv, [mol %] bez. auf eingesetztes Palladium | Reaktionstemperatur | Ausbeute Heck-Produkt in der Reaktionsmischung |
|---|---|---|---|
| 2.1 | ― ohne ― | 135° C | 92.9 % |
| 2.2 | Chrom(III)-acetylacetonat 20 mol % | 135°C | 95.0 % |
| 2.3 | Eisen(II)-acetat 20 mol % | 135° C | 96.7 % |
| 2.3 | Eisen(III)-acetylacetonat 10 mol % | 135° C | 96.9 % |
| 2.4 | Eisen(III)-chlorid Hexahydrat 10 mol % | 135°C | 97.4 % |
| 2.5 | Eisen(III)-chlorid Hexahydrat 20 mol % | 135°C | 97.7 % |
| | | | |
| 2.6 | Eisen(III)-chlorid Hexahydrat 200 mol % | 135°C | 97.1 % |
| 2.7 | Nickel(II)chlorid Hexahydrat 20 mol % | 135°C | 96.3 % |

### Beispiel 3:

Durchführung wie in Beispiel 1 beschrieben, wobei N,N-Dimethylglycin durch 140.2 mg (0.8 mmol) 3-Indolylessigsäure ersetzt wurde.

**Tabelle 3:**

| Beispiel Nr. | Metallhaltiges Additiv, [mol %] bez. auf eingesetztes Palladium | Reaktionstemperatur | Ausbeute Heck-Produkt in der Reaktionsmischung |
|---|---|---|---|
| 2.1 | ― ohne ― | 135° C | 90.3 % |
| 2.2 | Chrom(III)-acetylacetonat 20 mol % | 135°C | 94.6 % |
| 2.3 | Eisen(II)-acetat 20 mol % | 135° C | 97.0 % |
| 2.3 | Eisen(III)-acetylacetonat 10 mol % | 135° C | 96.8 % |
| 2.4 | Eisen(III)-chlorid Hexahydrat 10 mol % | 135°C | 97.0 % |
| 2.5 | Eisen(III)-chlorid Hexahydrat 20 mol % | 135°C | 97.1 % |
| 2.6 | Eisen(III)-chlorid Hexahydrat 200 mol % | 135°C | 96.7 % |
| 2.7 | Nickel(II)-chlorid Hexahydrat 20 mol % | 135°C | 95.1 % |

### Beispiel 4 - Vergleichsbeispiel gemäß DE 198 43 012

In einen 2 I Vierhalskolben mit KPG-Rührer, Rückflußkühler und Innenthermometer wurden 63.20 g (0.1 mol) 2,2',7,7'-Tetrabromspirobifluoren, 0.898 g (0.004 mol) Palladium(II)-acetat, 8.25 g (0.08 mol) N,N-Dimethylglycin, 201.62 g (2.4 mol) Natriumhydrogencarbonat und 108.15 g (0.60 mol) 1,1-Diphenylethen eingewogen und mit 500 ml N-Methylpyrrollidinon versetzt. Die Suspension wurde unter Rühren mit Stickstoff entgast, während einer Stunde auf 140°C Innentemperatur aufgeheizt und 18 h bei dieser Temperatur gehalten. Nach 18 Stunden ließ man auf 70°C abkühlen, setzte 500 ml Toluol und 300 ml Wasser zu, rührte 2 Stunden nach, trennte die Wasserphase ab und wusch die organische Phase dreimal mit 300 ml Wasser. Die organische Phase wurde in 1500 ml Methanol eingerührt. Der ausgefallene Niederschlag wurde abgesaugt und getrocknet

Die Ausbeute an dem Heck-Rohprodukt 2,2',7,7'-Tetrakis(2,2'-diphenylvinyl)-spiro-9,9'-bifluoren mit einer Reinheit von 93.8-94.3 % betrug bei 5 Wiederholungen jeweils zwischen 90 - 95 g (Rohausbeute: 87-92%).
Dieses Rohprodukt wurde jeweils (je nach Ansatz) 10 - 15 mal aus Dioxan umkristallisiert bis eine Reinheit von > 99.9 % erreicht war. Die Ausbeute an 2,2',7,7'-Tetrakis(2,2'-diphenylvinyl)-spiro-9,9'-bifluoren mit einer Reinheit von > 99.9 % betrug schließlich zwischen 25 - 35 g (Reinausbeute: 24-34%).

### Beispiel 5:

Durchführung wie in Beispiel 4 beschrieben, aber unter Zugabe von 20 mol % des metallhaltigen Additivs Eisen(III)-chlorid Hexahydrat bezogen auf das eingesetzte Palladium(II)-acetat, entsprechend 0.216 g (0.0008 mol).
Die Ausbeute an dem Heck-Rohprodukt 2,2',7,7'-Tetrakis(2,2'-diphenylvinyl)-spiro-9,9'-bifluoren mit einer Reinheit von 98.0-98.4 % betrug bei 5 Wiederholungen jeweils zwischen 95 -100 g (Rohausbeute: 92-97%).
Das Rohprodukt wurde jeweils (je nach Ansatz) 5 - 7 mal aus Dioxan umkristallisiert bis eine Reinheit von > 99.9 % erreicht war. Die Ausbeute an 2,2',7,7'-Tetrakis(2,2'diphenylvinyl)-spiro-9,9'-bifluoren mit einer Reinheit von > 99.9 % betrug schließlich zwischen 50 - 65 g (Reinausbeute: 49-63%).

### Beispiel 6- Vergleichsbeispiel gemäß DE 198 43 012

In einen 2 I Vierhalskolben mit KPG-Rührer, Rückflußkühler und Innenthermometer wurden 29.60 g (0.1 mol) 2,5-Dibrom-1,4-bis(hydroxymethyl)-benzol, 0.449 g (0.002 mol) Palladium(II)-acetat, 4.13 g (0.04 mol) N,N-Dimethylglycin, 50.41 g (0.6 mol) Natriumhydrogencarbonat und 54.08 g (0.30 mol) 1,1-Diphenylethen eingewogen und mit 250 ml N-Methylpyrrolidinon versetzt. Die Suspension wurde unter Rühren mit Stickstoff entgast, während einer Stunde auf 140°C Innentemperatur aufgeheizt und 12 h bei dieser Temperatur gehalten. Nach 18 Stunden ließ man auf 70°C abkühlen, setzte 500 ml Toluol und 500 ml Wasser zu, rührte 2 Stunden nach, saugte vom ausgefallenen, farblosen Feststoff ab und wusch diesen dreimal mit 200 ml Methanol nach.

Die Ausbeute an Heck-Rohprodukt 2,5-Bis(2,2'-diphenylvinyl)-1,4-bis(hydroxymethyl)-benzol mit einer Reinheit von 96.0-96.4 % betrug in drei Wiederholungsansätzen zwischen 40 - 43 g (Rohausbeute: 81-87%).
Das Rohprodukt wurde jeweils (je nach Ansatz) 7 - 9 mal aus Dichlormethan / Methanol (1/2 v/v) umkristallisiert bis eine Reinheit von > 99.8 % erreicht ist. Die Ausbeute an 2,5-Bis(2,2'-diphenylvinyl)-1,4-bis(hydroxymethyl)-benzol mit einer Reinheit von > 99.8 % betrug schließlich zwischen 20 - 25 g (Reinausbeute: 40-51%).

### Beispiel 7:

Durchführung wie in Beispiel 6 beschrieben, aber unter Zugabe von 10 mol % des metallhaltigen Additivs Eisen(III)-chlorid Hexahydrat bezogen auf die eingesetzte Palladium(II)-acetat-Menge, entsprechend 0.054 g (0.0002 mol).
Die Ausbeute an Heck-Rohprodukt 2,5-Bis(2,2'-diphenylvinyl)-1,4-bis(hydroxymethyl)-benzol mit einer Reinheit von 98.2-98.5 % betrug bei 3 Wiederholungsansätzen 44 - 47 g (Rohausbeute: 89-95%).
Das Rohprodukt wurde jeweils (je nach Ansatz) 3 - 5 mal aus Dichlormethan / Methanol (1/2 v/v) umkristallisiert bis eine Reinheit von > 99.8 % erreicht war. Die Ausbeute an 2,5-Bis(2,2'-diphenylvinyl)-1,4-bis(hydroxymethyl)-benzol mit einer Reinheit von > 99.8 % betrug schließlich zwischen 38-43 g (Reinausbeute: 77-87%).

### Beispiel 8 - Vergleichsbeispiel gemäß DE 198 43 012

In einen 2 I Vierhalskolben mit KPG-Rührer, Rückflußkühler und Innenthermometer wurden 81.87 g (100 mmol) 2,7-Dibrom-2',3',6',7'-tetrakis(2-methylbutyloxy)-spiro-9,9'-bifluoren, 0.225 g (1 mmol) Palladium(II)-acetat, 2.07 g (20 mmol) N,N-Dimethylglycin, 25.2 g (0.3 mol) Natriumhydrogencarbonat und 31.65 g (100 mmol) 1-(3,7-Dimethyloctyloxy)-4-methoxy-2,5-divinyl-benzol eingewogen und mit 1000 ml N-Methylpyrrolidinon versetzt. Die Suspension wurde unter Rühren mit Stickstoff entgast, während einer Stunde auf 140°C Innentemperatur aufgeheizt und 12 h bei dieser Temperatur gehalten. Während dieser Zeit erhöhte sich die Viskosität der Lösung deutlich. Danach wurde der Ansatz auf 70°C abgekühlt und auf 1000 ml Wasser gegeben. Das ausgefallene, gelbe Rohpolymer wurde abgesaugt, mit Methanol gewaschen und im Vakuumtrockenschrank getrocknet (Rohausbeute ∼97 g, d. h. ∼100%).
Die Aufreinigung erfolgte durch zweimaliges Lösen in THF und jeweiliges Ausfällen in Methanol. Die Lösungen wurden dabei jeweils 10%ig angesetzt.
Die Ausbeute an Poly[(2',3',6',7'-tetrakis(2-methylbutyloxy)-spiro-9,9'-bifluoren-2,7-ylen-1,2-vinylen)-*alt*-({2-(3,7-dimethyloctyloxy)-5-methoxy-1,4-phenylen}-1,2-vinylen)] nach diesen Aufreinigungsschritten betrug 77.0 g (79%). Das Polymer ist in Lösemitteln wie Dichlormethan, THF, Toluol gut löslich, hingegen in Alkoholen wie Methanol, Ethanol praktisch unlöslich und ein pulveriger gelber Feststoff.
GPC: THF + 0.25% Oxals.; Säulensatz SDV500, SDV1000, SDV10000 (Fa. *PSS*), 35°C, RI-Detektion, Polystyrol-Standard: M_{w} = 6.5×10⁴ g/mol, Mₙ = 2.9×10⁴ g/mol (entspricht einem Polymerisationsgrad DP ≈ 30).

### Beispiel 9:

Durchführung wie in Beispiel 8 beschrieben, aber unter Zugabe von 10 mol % des metallhaltigen Additivs Eisen(III)-chlorid Hexahydrat bezogen auf die eingesetzte Palladium(II)-acetat-Menge, entsprechend 0.027g (0.1 mmol).
Die Aufarbeitung erfolgte ebenfalls analog Beispiel 8, allerdings konnten beim Umfällen hier wegen der höheren Viskositäten nur 6%ige Lösungen in THF verwendet werden.
Die Ausbeute am Polymer analog Beispiel 8 nach diesen Aufreinigungsschritten betrug 81.4 g (84%). Das Polymer ist ein fasriger gelber Feststoff.
GPC: THF + 0.25% Oxals.; Säulensatz SDV500, SDV1000, SDV10000 (Fa. *PSS*), 35°C, RI-Detektion, Polystyrol-Standard: M_{w} = 3.1×10⁵ g/mol, Mₙ = 1.45×10⁵ g/mol (entspricht einem Polymerisationsgrad DP ≈ 150).

## Patentansprüche

1. Verfahren zur Umsetzung einer funktionellen Aryl- oder Heteroarylverbindung mit einem Olefinderivat, welches mindestens ein H-Atom an der Doppelbindung aufweist, in Gegenwart einer einfachen Palladiumverbindung, gegebenenfalls in der Gegenwart eines stickstoffhaltigen Additives, einer Base in einem Lösungsmittel, unter Bildung einer C-C-Verknüpfung und formaler Abspaltung der funktionellen Gruppe des Aryl- bzw. Heteroarylderivates und eines H-Atoms der Olefinverbindung, **gekennzeichnet durch** die Anwesenheit von mindestens einem metallhaltigen Additiv, das von Palladium verschieden ist, in einer Menge zwischen 1 und 500 mol% (bezogen auf die Palladiummenge).

2. Verfahren gemäß Anspruch 1, **dadurch gekennzeichnet daß** ein stickstoffhaltiges Additiv verwendet wird.

3. Verfahren gemäß den Ansprüchen 1 oder 2, **dadurch gekennzeichnet, daß** das stickstoffhaltige Additiv eine Aminocarbonsäure bzw. ein Aminocarbonsäurederivat ist.

4. Verfahren gemäß mindestens einem der Ansprüche 1 bis 3, **dadurch gekennzeichnet, daß** das stickstoffhaltige Additv Dimethylglycin, 4-Dimethylaminobuttersäure oder 3-Indolylessigsäure ist.

5. Verfahren gemäß mindestens einem der Ansprüche 1 bis 4, **dadurch gekennzeichnet, daß** das metallhaltige Additiv aus Metallen, Legierungen, Verbindungen, Salzen oder Chalkogeniden der d-Übergangsgruppen des Periodensystems ausgewählt ist.

6. Verfahren gemäß Anspruch 5, **dadurch gekennzeichnet, daß** das metallhaltige Additiv Übergangsmetalle ab der Vanadiumgruppe bis zur Zinkgruppe enthält.

7. Verfahren gemäß den Ansprüchen 5 oder 6, **dadurch gekennzeichnet, daß** das metallhaltige Additiv mindestens ein Metall ausgewählt aus Eisen, Kobalt, Nickel, Ruthenium, Rhodium, Osmium, Iridium oder Platin enthält.

8. Verfahren gemäß mindestens einem der Ansprüche 5 bis 7, **dadurch gekennzeichnet, daß** das metallhaltige Additiv mindestens ein Metall ausgewählt aus Eisen, Kobalt oder Nickel enthält.

9. Verfahren gemäß mindestens einem der Ansprüche 5 bis 8, **dadurch gekennzeichnet, daß** das metallhaltige Additiv Eisen enthält.

10. Verfahren gemäß mindestens einem der Ansprüche 5 bis 9, **dadurch gekennzeichnet, daß** als metallhaltige Additive Halogenide, Sulfate, Acetate oder weitere Carboxylate eingesetzt werden.

11. Verfahren gemäß mindestens einem der Ansprüche 5 bis 9, **dadurch gekennzeichnet, daß** als metallhaltige Additive Oxide oder Komplexe eingesetzt werden.

12. Verfahren gemäß mindestens einem der Ansprüche 5 bis 9, **dadurch gekennzeichnet, daß** als metallhaltige Additive Metalle oder metallische Legierungen eingesetzt werden.

13. Verfahren gemäß Anspruch 9, **dadurch gekennzeichnet, daß** als eisenhaltiges Additiv Eisenerze, verschiedene Stahl- oder Gußeisenformen eingesetzt werden.

14. Verfahren gemäß mindestens einem der vorhergehenden Ansprüche, **dadurch kennzeichnet, daß** multifunktionelle Aryl- bzw. Heteroarylverbindungen und monofunktionell reagierende Olefine eingesetzt werden.

15. Verfahren gemäß mindestens einem der Ansprüche 1 bis 13, **dadurch kennzeichnet, daß** monofunktionelle Aryl- bzw. Heteroarylverbindungen und multifunktionell reagierende Olefine bzw. Verbindungen, die mehrere monofunktionelle Olefinenden besitzen, eingesetzt werden.

16. Verfahren gemäß mindestens einem der Ansprüche 1 bis 13, **dadurch kennzeichnet, daß** Polymere durch die Umsetzung von bifunktionellen Aryl- bzw. Heteroarylverbindungen mit bifunktionellen Olefinderivaten hergestellt werden.

17. Verfahren gemäß mindestens einem der Ansprüche 1 bis 14 zur Herstellung von 2,2',7,7'-Tetrakis(2,2'-diphenylvinyl)-spiro-9,9'-bifluoren.

## Claims

1. A process for reacting a functional aryl or heteroaryl compound with an olefin derivative which has at least one hydrogen atom on the double bond in the presence of a simple palladium compound, optionally in the presence of a nitrogen additive, and of a base in a solvent, to form a C-C bond and formally cleave off the functional group of the aryl or heteroaryl derivative and a hydrogen atom of the olefin compound, **characterized by** the presence of at least one metal additive other than palladium.

2. The process as claimed in claim 1, **characterized in that** a nitrogen additive is used.

3. The process as claimed in claim 1 or 2, **characterized in that** the nitrogen additive is an aminocarboxylic acid or an aminocarboxylic acid derivative.

4. The process as claimed in at least one of claims 1 to 3, **characterized in that** the nitrogen additive is dimethylglycine, 4-dimethylaminobutyric acid or 3-indolylacetic acid.

5. The process as claimed in at least one of claims 1 to 4, **characterized in that** the metal additive is selected from metals, alloys, compounds, salts or chalcogenides of the d transition groups of the Periodic Table.

6. The process as claimed in claim 5, **characterized in that** the metal additive comprises transition metals from the vanadium group to the zinc group.

7. The process as claimed in claim 5 or 6, **characterized in that** the metal additive comprises at least one metal selected from iron, cobalt, nickel, ruthenium, rhodium, osmium, iridium and platinum.

8. The process as claimed in at least one of claims 5 to 7, **characterized in that** the metal additive comprises at least one metal selected from iron, cobalt and nickel.

9. The process as claimed in at least one of claims 5 to 8, **characterized in that** the metal additive comprises iron.

10. The process as claimed in at least one of claims 5 to 9, **characterized in that** the metal additives used are halides, sulfates, acetates or other carboxylates.

11. The process as claimed in at least one of claims 5 to 9, **characterized in that** the metal additives used are oxides or complexes.

12. The process as claimed in at least one of claims 5 to 9, **characterized in that** the metal additives used are metals or metallic alloys.

13. The process as claimed in claim 9, **characterized in that** the iron additive used is an iron ore, or various forms of steel or cast iron.

14. The process as claimed in at least one of the preceding claims, **characterized in that** multifunctional allyl or heteroaryl compounds and olefins which react monofunctionally are used.

15. The process as claimed in at least one of claims 1 to 13, **characterized in that** monofunctional aryl or heteroaryl compounds and olefins or compounds which have a plurality of monofunctional olefinic ends and react multifunctionally are used.

16. The process as claimed in at least one of claims 1 to 13, **characterized in that** polymers are prepared by reacting bifunctional aryl or heteroaryl compounds with bifunctional olefin derivatives.

17. The process as claimed in at least one of claims 1 to 14 for preparing 2,2',7,7'-tetrakis(2,2'-diphenylvinyl)spiro-9,9'-bifluorene.

## Revendications

1. Procédé pour faire réagir un composé aryle ou hétéroaryle fonctionnel avec un dérivé d'oléfine qui comporte au moins un atome d'hydrogène sur la double liaison, en présence d'un composé du palladium simple, éventuellement en présence d'un additif azoté, d'une base dans un solvant, avec formation d'une liaison C-C et clivage formel du groupe fonctionnel du dérivé aryle ou hétéroaryle et d'un atome d'hydrogène du composé d'oléfine, **caractérisé par** la présence d'au moins un additif contenant des métaux, qui est différent du palladium, en une quantité entre 1 et 500 mol% (par rapport à la quantité de palladium).

2. Procédé selon la revendication 1 **caractérisé en ce qu'**un additif azoté est utilisé.

3. Procédé selon les revendications 1 ou 2 **caractérisé en ce que** l'additif azoté est un acide aminocarboxylique ou un dérivé d'acide aminocarboxylique.

4. Procédé selon au moins l'une des revendications 1 à 3 **caractérisé en ce que** l'additif azoté est la diméthylglycine, l'acide 4-diméthylaminobutyrique ou l'acide 3-indolylacétique.

5. Procédé selon au moins l'une des revendications 1 à 4 **caractérisé en ce que** l'additif contenant des métaux est choisi parmi les métaux, les alliages, les composés, les sels ou les chalcogénures des groupes de transition d du système périodique.

6. Procédé selon la revendication 5 **caractérisé en ce que** l'additif contenant des métaux contient des métaux de transition du groupe du vanadium au groupe du zinc.

7. Procédé selon les revendications 5 ou 6 **caractérisé en ce que** l'additif contenant des métaux contient au moins un métal choisi parmi le fer, le cobalt, le nickel, le ruthénium, le rhodium, l'osmium, l'iridium ou le platine.

8. Procédé selon au moins l'une des revendications 5 à 7 **caractérisé en ce que** l'additif contenant des métaux contient au moins un métal choisi parmi le fer, le cobalt ou le nickel.

9. Procédé selon au moins l'une des revendications 5 à 8 **caractérisé en ce que** l'additif contenant des métaux contient du fer.

10. Procédé selon au moins l'une des revendications 5 à 9 **caractérisé en ce que** des halogénures, des sulfates, des acétates ou d'autres carboxylates sont utilisés comme additifs contenant des métaux.

11. Procédé selon au moins l'une des revendications 5 à 9 **caractérisé en ce que** des oxydes ou des complexes sont utilisés comme additifs contenant des métaux.

12. Procédé selon au moins l'une des revendications 5 à 9 **caractérisé en ce que** des métaux ou des alliages métalliques sont utilisés comme additifs contenant des métaux.

13. Procédé selon la revendication 9 **caractérisé en ce que** des minerais de fer, différentes formes d'acier ou de fonte sont utilisés comme additif contenant du fer.

14. Procédé selon au moins l'une des revendications précédentes **caractérisé en ce que** des composés aryle ou hétéroaryle multifonctionnels et des oléfines réagissant de manière monofonctionnelle sont utilisés.

15. Procédé selon au moins l'une des revendications 1 à 13 **caractérisé en ce que** des composés aryle ou hétéroaryle monofonctionnels et des oléfines réagissant de manière multifonctionnelle ou des composés qui possèdent plusieurs extrémités d'oléfines monofonctionnelles sont utilisés.

16. Procédé selon au moins l'une des revendications 1 à 13 **caractérisé en ce que** des polymères sont produits par la réaction de composés aryle ou hétéroaryle bifonctionnels avec des dérivés d'oléfines bifonctionnels.

17. Procédé selon au moins l'une des revendications 1 à 14 pour la production du 2,2',7,7'-tétrakis(2,2'-diphénylvinyl)-spiro-9,9'-bifluorène.
